# EUROPEAN PATENT SPECIFICATION

(11) **EP 3 808 318 B1**
(45) Date of publication and mention of the grant of the patent: **29.06.2022**
(21) Application number: 20200776.1
(22) Date of filing: 08.10.2020
(51) Int. Cl.: A61F 5/01

(54) **ARTICULATED JOINT FOR ORTHOPAEDIC ORTHOSES OR BRACES**
ARTIKULIERTE VERBINDUNG FÜR ORTHOPÄDISCHE ORTHESEN
JOINT ARTICULÉ POUR ORTHÈSES ORTHOPÉDIQUES

(30) Priority: 18.10.2019 IT 201900019298
(43) Date of publication of application: 21.04.2021
(73) Proprietor: F.G.P. S.R.L., 37062 Villafranca Di Verona (Verona) (IT)
(72) Inventor: FERRIGOLO, Moreno, 37062 Villafranca di Verona (IT); TURRINI, Alberto, 37062 Villafranca di Verona (IT)
(74) Representative: Sandri, Sandro

(56) References cited:
- EP-A1- 1 475 060
- EP-A1- 1 832 255
- WO-A1-2005/039459
- WO-A1-2011/137999
- US-A- 6 090 057

## Description

### FIELD OF APPLICATION

The present invention relates to an articulated joint for a pelvic bar or for other components of orthopaedic orthoses or braces designed for supporting the torso, such as spinal hyperextension braces, or, more in general, for the rehabilitation of joints of the human body, such as the knee, elbow, ankle, hip, shoulder or the like.

The present solution envisages the manufacture of a "simplified" articulated joint positioned at the junction of two bars, said articulated joint comprising a selector that enables the selection of the angular position of one bar relative to the other. Said selector is represented by a vertical slide sliding in a specific guide acting as a locking element which can be actuated by the wearer, since it functions without the use of accessories and consequently acts in a simplified manner, also without the aid of personnel, thus enabling the wearer to actuate it manually.

The present invention has application in the medical and orthopaedic industry and in particular in the production of braces in general, as well as of orthoses and braces mainly utilisable in conservative, post-traumatic, rehabilitative and post-operative therapy.

### PRIOR ART

It is known in the orthopaedic sector that subjects presenting with orthopaedic problems either of the spinal column or of joints such as the knee, ankle or elbow, especially in the case of injuries or as a consequence of a previous surgical intervention, need to use an orthopaedic brace, or orthosis, designed to ensure or control the function of supporting the spinal column or providing a hinged constraint between the femur and tibia or other lever pivot points of joints, by withstanding stresses that would otherwise be damaging for the joint itself.

The function of an orthosis is, in general, to ensure a relative immobilisation or limitation of the torso or of a joint affected by injuries or arthritis or ligament sprains or which has undergone a surgical procedure.

Another use of orthoses is in conjunction with functional rehabilitation or re-education, where the orthosis can be used to reduce the load on a joint and decrease its pain, or be adopted for preventive purposes in cases of osteoporosis or bone yielding.

In the specific case of problems of an orthopaedic type affecting the spinal column or torso of persons subject, for example, to osteoporosis or other forms, both degenerative and inflammatory, or also of traumatic origin, it is necessary to use orthopaedic braces or orthoses of the corset type, which ensure a certain support for the patient by absorbing the most intense stresses that develop in the torso.

Remaining in the specific sector of problems of the torso or spinal column, though the concept at the basis of the invention can be applied to all orthoses, various types of corsets, braces or orthoses designed to support or contain the torso are currently known and available.

These are generally structures that rest against the spinal column and consist for the most part of a plate for stiffening the spinal column, and thus shaped with the same conformation, which is attached by using special straps so as to be held in place on the torso.

According to other solutions, dorsal orthoses normally consist of a rigid structure, on which areas of padding are disposed, and consisting of two lateral uprights associated at the top with a sternal plate and connected at the bottom, by means of articulated joints, to a tilting pelvic band.

In the latter case, the tilting pelvic band is connected to the two lateral uprights by means of articulated joints that are generally of the freely rotating type or have adjustable-angle constraint means of complex conception, in that they allow the desired positioning angle to be set through complex mechanisms that can only be used by the personnel assigned to control the orthosis.

Also known is the presence, on some braces present in the market, of particular retaining systems for which use is made of screws or pins that are inserted in specific seats of the articulated joint and are not parts integrated into the orthoses, making both their production and adjustment complex and impractical.

Furthermore, the use of such screws or pins used in some traditional braces can be rather uncomfortable or even impossible for the wearer and require adequate support from specialised personnel, who must use mechanical adjustment equipment.

It may further be observed that, in the case of a freely rotating or spring-opposed articulated joint, the pelvic band can take on any position, thus rendering the orthosis unstable or subject to phenomena of jamming that make it impractical and uncomfortable for patients who wear them, for whom it would rather be desirable to lock the oscillation of the tilting part in the desired position by acting on a selector with a simple movement performed with one's fingers, without having to rely on personnel.

These critical issues, which have been highlighted in the case of articulated joints present on orthoses for the upper torso or torso, have also been noted in the case of mechanical articulated joints of other types, such as those used laterally to the femur and to the tibia, in the case of an example of application to the lower limb, or articulated joints applied on the elbow or ankle or joints of any other kind, which are thus subject to the same problems.

Document WO2005/039459 A1 discloses an articulated joint having the features described in the preamble of claim 1.

### DESCRIPTION OF THE INVENTION

The present invention aims to provide an articulated joint for orthopaedic orthoses or braces designed for rehabilitation or stabilisation both of the torso and of the knee, or other orthopaedic braces applicable as supports for joints of the human body, such as the ankle, the elbow or the like, and which is capable of eliminating or at least reducing the drawbacks highlighted above.

This aim is achieved by using an articulated joint for orthopaedic orthoses or braces designed for rehabilitation or stabilisation of the torso or joints in general which is made with a conception aimed at the possibility of locking the tilting pelvic bar in the top dead centre of the angle of movement, for example to make it easier for the patient to sit during the period of use.

The brace according to the invention, unlike traditional ones, possesses an integrated adjustment element for adjusting the articulated joint, consisting of a small slide positioned in a special guide which, when the pelvic bar reaches the top dead centre, can be pushed downward so as to be inserted into a seat and block the rotation of the articulated joint.

The slide, which is present in both articulated joints, has two clicks mechanisms that identify and lock the open and closed positions.

This is obtained by means of an articulated joint for orthopaedic orthoses or braces designed for rehabilitation or stabilisation of the spinal column or of a joint, whose features are described in the main claim.

The dependent claims of the solution in question outline advantageous embodiments of the invention.

The main advantages of this solution regard first of all the fact that the articulated joint as a whole, compared to the known solutions, features simplified components comprising a simple position selector that can be actuated at the appropriate moment without the use of accessories and/or without the aid of personnel, as it is the user who actuates it manually.

### ILLUSTRATION OF THE DRAWINGS

Other features and advantages of the invention will become more apparent on reading the following description of one embodiment of the invention, provided by way of non-limiting example with the aid of the drawings illustrated in the attached figures, in which:
- Figure 1 represents a schematic front view of the articulated joint according to the invention applied to an orthosis for the torso, where it is shown that the pelvic bar of the brace possesses a tilting movement with an elastic return;
- Figure 2 shows the same schematic front view of the articulated joint according to the invention, where it is shown that the tilting movement with an elastic return occurs if the locking selector is not inserted;
- Figure 3 illustrates the same schematic front view of the articulated joint according to the invention, where it is shown that the pelvic bar can be locked in the highest part of the arc in the tilting phase by manually actuating the locking selector, which is inserted in the seat provided in the articulated joint;

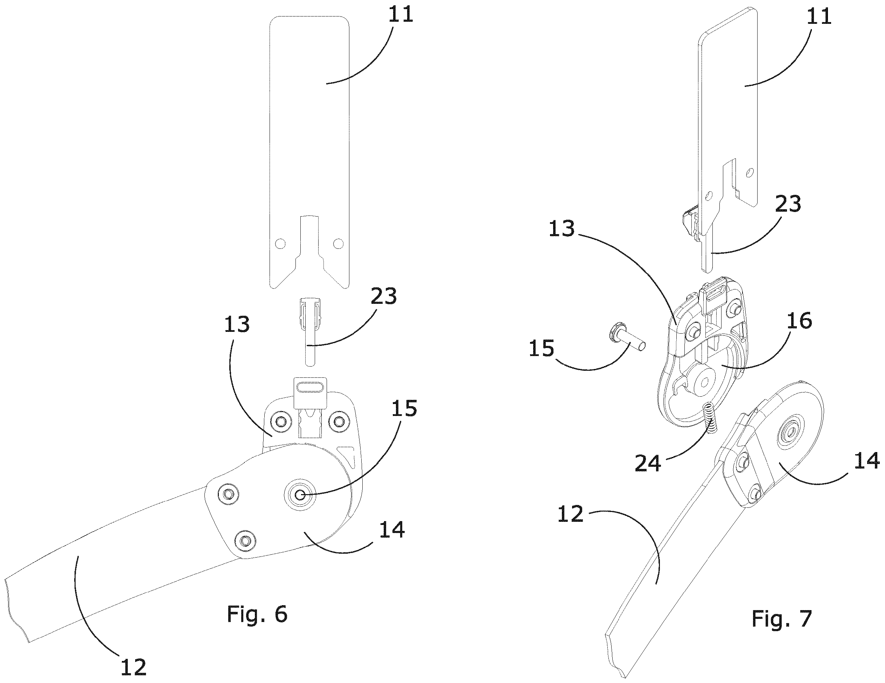

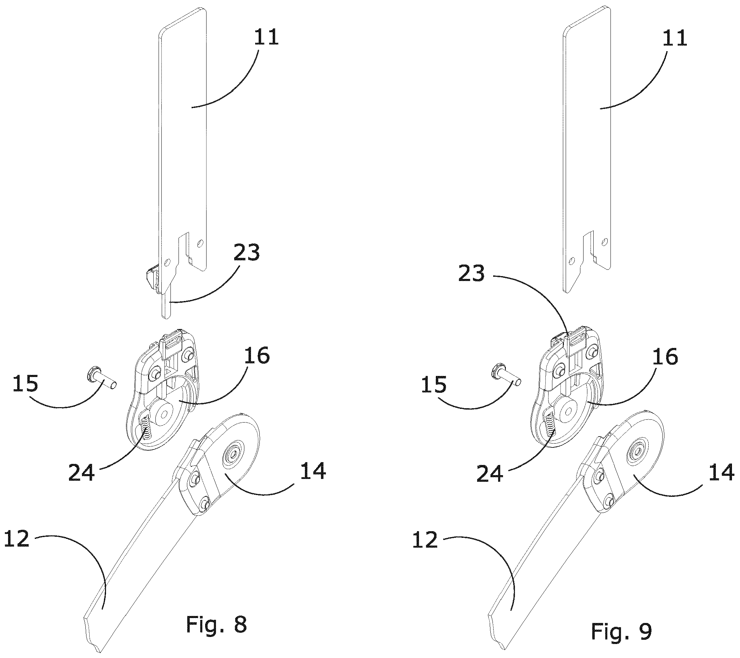

- Figures 4 to 9 represented exploded schematic views showing the positions of the various components in different assembly steps.

### DESCRIPTION OF AN EMBODIMENT OF THE INVENTION

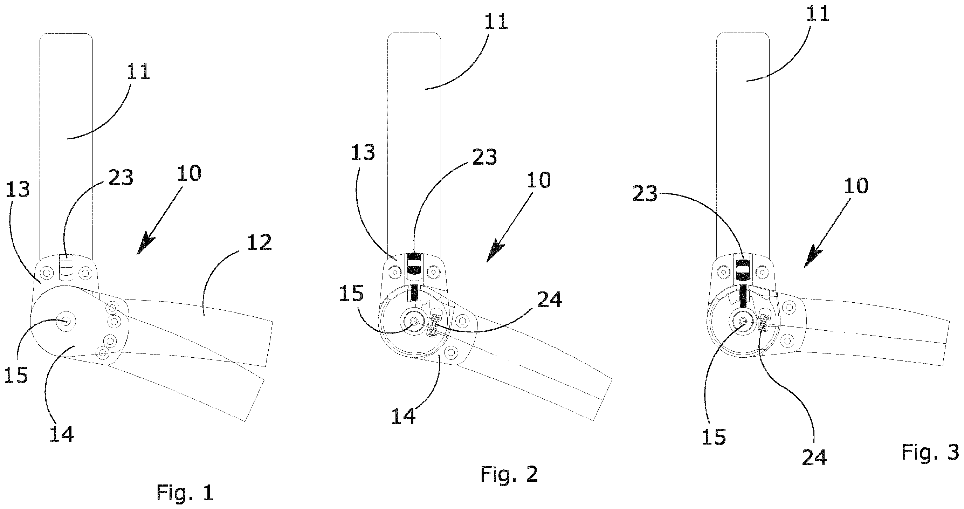

Making reference initially to figure 1, the number 10 denotes in its entirety an articulated joint for orthopaedic orthoses or braces designed for rehabilitation or stabilisation of the torso, or for orthopaedic orthoses or braces applicable on joints of the body in general; it is made with a conception aimed at the possibility of locking one of the tilting pelvic bars as opposed to the other, that is to say, for orthoses of the spinal cord, to lock the tilting pelvic bar in the top dead centre of the angle of movement, for example to make it easier for the patient to sit during the period of use.

The articulated joint 10 according to the invention is used for an angularly adjustable connection between a first bar 11 and a second bar 12, which are part of a rehabilitative or stabilising orthosis for orthopaedic use.

The articulated joint according to the invention is composed of two half parts 13 and 14 reciprocally connected and retained by a common rotation pin 15, each of which, at opposite ends relative to the rotation pin, is stably associated with each end of the respective bars 11 and 12, so as to create a jointed mechanism applicable in the point of the orthosis that requires an adjustable angular movement.

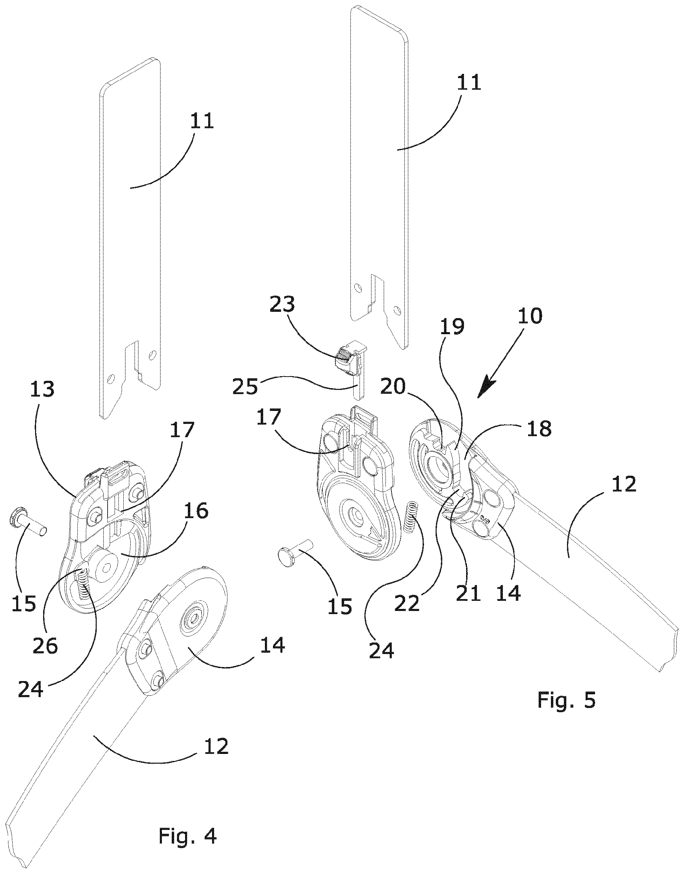

As represented in figures 4 and 5, said two reciprocally connected half parts 13 and 14 retained by a common rotation pin 15 comprise grooves disposed on the inner side thereof facing towards the inner side of the opposite part, said grooves being reciprocally offset in such a manner as to enable the reciprocal penetration between the two half parts 13 and 14.

More precisely, the first half part 13 is provided with a semi-circular groove 16 and a seat 17 disposed in a radial direction, whilst the second half part 14 is provided with a groove 18 which delimits a first tooth 19, in turn adapted to define a seat 20, which, during use, is disposed on the same axis in the seat 17 present in the first half part 13, and a second tooth 21 adapted to define a seat 22.

Furthermore, the articulated joint according to the invention comprises a slidable selector 23 and at least one spring 24.

The slidable selector 23 comprises a stem 25 adapted to be inserted, during use, into said seat 17 of the first half part 13 positioned in alignment with said seat 20 of the second half part 14, whereas at least one spring 24 is inserted in at least one housing 26 fashioned from one end of said semi-circular groove 16 of the first half part 13.

It is envisaged that the springs and the respective housings can be two, if necessary, in order to facilitate, in other words, to render more efficient, the elastic thrust during the steps of adjusting the articulated joint.

From an operational viewpoint, the first bar 11 is fixed to the first half part 13 and, likewise, the second bar 12 is fixed to the second half part 14.

At this point the first half part 13 is connected with the second half part 14 after the insertion of the spring 24 into the housing 26, so that the spring itself remains comprised between the housing 26 and the seat 22 defined by said second tooth 21.

The pin 15 maintains the two half parts 13 and 14 connected, and once the two half parts are reciprocally assembled, it is possible to introduce the stem 25 of the selector 23 into the seat 17, so that it is disposed according to an open or closed mode relative to said seat 20 fashioned in the second half part 14.

The selector 23 can undergo a movement in order to be brought from an open position to a closed position relative to said seat 20, so as to enable or prevent, respectively, the angular rotation of the second bar 12 relative to the first bar 11.

In particular, when the selector 23 is in the open position, it remains raised relative to said seat 20 of the second half part 14, thus enabling the free reciprocal rotation between the two half part 13 and 14 and, consequently, between the bars 11 and 12, a rotation that is of an elastic type in that it acts in opposition to the thrust exerted by the spring 24.

In order to bring the selector 23 into the closed position, the selector is manually moved into the seat 17, penetrating into the latter until reaching the seat 20, which brings about a locking condition due to the opposition with the tooth 19, which prevents the angular movement between the two half parts.

As may be noted, the articulated joint according to the invention, unlike traditional articulated joints, possesses an integrated adjustment element for adjusting the articulated joint that allows the rotation of the bars to be blocked when the lower bar reaches the top dead centre, simply by pushing it downward, thus enabling the insertion thereof into a seat 20, which will block the rotation of the articulated joint.

The invention has been described above with reference to a preferred embodiment thereof. However, it is clear that the invention is susceptible of numerous variants falling within the scope thereof, within the framework of technical equivalences.

## Claims

1. An articulated joint (10) for orthopaedic orthoses or braces designed for rehabilitation or stabilisation of the spinal column or joints of the body in general, said articulated joint comprising two half parts (13, 14) reciprocally connected and retained by a common rotation pin (15), each of which, at opposite ends relative to the rotation pin, is stably associated with each end of respective bars (11, 12), so as to create a jointed mechanism applicable in the point of the orthosis that requires an adjustable angular movement, wherein said two half parts (13, 14) comprise grooves disposed on the inner side thereof facing towards the inner side of the opposite part, said grooves being reciprocally offset in such a manner as to enable the reciprocal penetration between the two half parts (13, 14) and wherein at least one (13) of said two half parts comprises a seat (17) into which a selector (23) provided with a stem (25) penetrates, the stem being moved from an unlocking position to a locking one in which it penetrates into a seat (22) of the second half part (14), respectively allowing or blocking the angular rotation of one half part (13) relative to the other (14), i.e. of one bar (11) relative to the other (12), in opposition to one or more springs (24), **characterised in that** said first half part (13) is provided with a semi-circular groove (16) and a seat (17) disposed in a radial direction, whilst the second half part (14) is provided with a groove (18) which delimits a first tooth (19), in turn adapted to define a seat (20), which, during use, is disposed on the same axis in the seat (17) present in the first half part (13), and a second tooth (21) adapted to define a seat (22) .

2. The articulated joint (10) for orthopaedic orthoses or braces according to claim 1, **characterised in that** it comprises a slidable selector (23) which is insertable into said seat (17) so as to intercept said seat (22) of the second half part (14).

3. The articulated joint (10) for orthopaedic orthoses or braces according to one of claims 1-2, **characterised in that** said slidable selector (23) is provided with a stem (25) adapted to be inserted, during use, into said seat (17) of the first half part (13) positioned in alignment with said seat (20) of the second half part (14).

4. The articulated joint (10) for orthopaedic orthoses or braces according to one of claims 1-3, **characterised in that** it comprises at least one spring (24) which is insertable in a housing (26) fashioned from one end of said semi-circular groove (16) of the first half part (13) .

5. The articulated joint (10) for orthopaedic orthoses or braces according to one of claims 1-4, **characterised in that** said selector (23) can be moved so as to be brought from an open position to a closed position relative to said seat (20), so as to enable or prevent, respectively, the angular rotation of the second bar (12) relative to the first bar (11).

6. The articulated joint (10) for orthopaedic orthoses or braces according to one of claims 1-5, **characterised in that** when the selector (23) is in the open position, it remains raised relative to said seat (20) of the second half part (14), enabling the free reciprocal rotation between the two half parts (13, 14) and consequently between the bars (11, 12).

7. The articulated joint (10) for orthopaedic orthoses or braces according to one of claims 1-6, **characterised in that** the selector (23) can be moved into a closed position in the seat (17), penetrating into the latter until reaching the seat (20) of the second half part (14), the locking being obtained due to the opposition between the selector (23) and the tooth (19) which prevents the angular movement between the two half parts.

8. The articulated joint (10) for orthopaedic orthoses or braces according to one of claims 1-7, **characterised in that** the rotation between the two half parts (13, 14), i.e. between the two bars (11, 12), is of an elastic type, since it is carried out in opposition to the thrust exerted by one or more springs (24).

## Patentansprüche

1. Gegliedertes Gelenk (10) für orthopädische Orthesen oder Stützen, die für eine Rehabilitation oder Stabilisierung der Wirbelsäule oder von Gelenken des Körpers im Allgemeinen ausgelegt sind, wobei das gegliederte Gelenk zwei Hälften (13, 14) umfasst, die durch einen gemeinsamen Drehzapfen (15) wechselseitig verbunden und gehalten werden, deren jede, an im Verhältnis zu dem Drehzapfen entgegengesetzten Enden, stabil mit jedem Ende jeweiliger Schienen (11, 12) verknüpft ist, um so einen gegliederten Mechanismus zu schaffen, der in dem Punkt der Orthese anwendbar ist, der eine einstellbare Winkelbewegung erfordert, wobei die zwei Hälften (13, 14) Rillen umfassen, die auf der inneren Seite derselben angeordnet sind, die hin zu der inneren Seite des gegenüberliegenden Teils zeigen, wobei die Rillen auf eine solche Weise wechselseitig versetzt sind, dass die wechselseitige Durchdringung zwischen den zwei Hälften (13, 14) ermöglicht wird, und wobei mindestens eine (13) der zwei Hälften einen Sitz (17) umfasst, in den ein Wählschalter (23) eindringt, der mit einem Schaft (25) versehen ist, wobei der Schaft von einer entriegelnden Stellung zu einer verriegelnden bewegt wird, in der er in einen Sitz (22) der zweiten Hälfte (14) eindringt, was jeweils die Winkeldrehung der einen Hälfte (13) im Verhältnis zu der anderen (14), d. h. einer Schiene (11) im Verhältnis zu der anderen (12), ermöglicht beziehungsweise sperrt, **dadurch gekennzeichnet, dass** die erste Hälfte (13) mit einer halbkreisförmigen Rille (16) und einem Sitz (17), der in einer radialen Richtung angeordnet ist, versehen ist, während die zweite Hälfte (14) mit einer Rille (18) versehen ist, die einen ersten Zahn (19), der wiederum angepasst ist, um einen Sitz (20) zu definieren, der, während der Anwendung, auf der gleichen Achse angeordnet ist wie der Sitz (17), der in der ersten Hälfte (13) vorhanden ist, und einen zweiten Zahn (21), der angepasst ist, um einen Sitz (22) zu definieren, begrenzt.

2. Gegliedertes Gelenk (10) für orthopädische Orthesen oder Stützen nach Anspruch 1, **dadurch gekennzeichnet, dass** es einen verschiebbaren Wählschalter (23) umfasst, der in den Sitz (17) einsteckbar ist, um so den Sitz (22) der zweiten Hälfte (14) aufzuhalten.

3. Gegliedertes Gelenk (10) für orthopädische Orthesen oder Stützen nach einem der Ansprüche 1 bis 2, **dadurch gekennzeichnet, dass** der verschiebbare Wählschalter (23) mit einem Schaft (25) versehen ist, der angepasst ist, um, während der Anwendung, in den Sitz (17) der ersten Hälfte (13) eingesteckt zu werden, der in Ausrichtung mit dem Sitz (20) der zweiten Hälfte (14) angeordnet ist.

4. Gegliedertes Gelenk (10) für orthopädische Orthesen oder Stützen nach einem der Ansprüche 1 bis 3, **dadurch gekennzeichnet, dass** es mindestens eine Feder (24) umfasst, die in ein Gehäuse (26) eingesteckt werden kann, das von einem Ende der halbkreisförmigen Rille (16) der ersten Hälfte (13) geformt wird.

5. Gegliedertes Gelenk (10) für orthopädische Orthesen oder Stützen nach einem der Ansprüche 1 bis 4, **dadurch gekennzeichnet, dass** der Wählschalter (23) so bewegt werden kann, dass er von einer offenen Stellung zu einer geschlossenen Stellung im Verhältnis zu dem Sitz (20) gebracht werden kann, um so jeweils die Winkeldrehung der zweiten Schiene (12) im Verhältnis zu der ersten Schiene (11) zu ermöglichen beziehungsweise zu verhindern.

6. Gegliedertes Gelenk (10) für orthopädische Orthesen oder Stützen nach einem der Ansprüche 1 bis 5, **dadurch gekennzeichnet, dass**, wenn sich der Wählschalter (23) in der offenen Stellung befindet, er im Verhältnis zu dem Sitz (20) der zweiten Hälfte (14) erhöht bleibt, was die freie wechselseitige Drehung zwischen den zwei Hälften (13, 14) und demzufolge zwischen den Schienen (11, 12) ermöglicht.

7. Gegliedertes Gelenk (10) für orthopädische Orthesen oder Stützen nach einem der Ansprüche 1 bis 6, **dadurch gekennzeichnet, dass** der Wählschalter (23) in eine geschlossene Stellung in dem Sitz (17) bewegt werden kann, wobei er in den letzteren eindringt, bis er den Sitz (20) der zweiten Hälfte (14) erreicht, wobei die Verriegelung auf Grund des Widerstandes zwischen dem Wählschalter (23) und dem Zahn (19) erreicht wird, was die Winkelbewegung zwischen den zwei Hälften verhindert.

8. Gegliedertes Gelenk (10) für orthopädische Orthesen oder Stützen nach einem der Ansprüche 1 bis 7, **dadurch gekennzeichnet, dass** die Drehung zwischen den zwei Hälften (13, 14), d. h., zwischen den zwei Schienen (11, 12), von einer elastischen Art ist, da sie in Widerstand zu dem Schub ausgeführt wird, der durch eine oder mehrere Federn (24) ausgeübt wird.

## Revendications

1. Articulation (10) pour orthèses ou attelles orthopédiques conçues pour la rééducation ou la stabilisation de la colonne vertébrale ou d'articulations du corps en général, ladite articulation comprenant deux moitiés (13, 14) reliées l'une à l'autre et retenues par une tige de rotation commune (15), dont chacune, à des extrémités opposées par rapport à la tige de rotation, est associée de manière stable à chaque extrémité de barres respectives (11, 12) pour créer un mécanisme articulé applicable au point de l'orthèse qui demande un mouvement angulaire réglable, dans laquelle lesdites deux moitiés (13, 14) comprennent des rainures disposées de leur côté intérieur en vis-à-vis du côté intérieur de la partie opposée, lesdites rainures étant décalées l'une par rapport à l'autre de manière à permettre la pénétration des deux moitiés (13, 14) l'une dans l'autre et dans laquelle au moins l'une (13) desdites deux moitiés comprend un siège (17) dans lequel pénètre un sélecteur (23) pourvu d'une tige (25), la tige étant déplacée d'une position de déverrouillage à une position de verrouillage dans laquelle elle pénètre dans un siège (22) de la seconde moitié (14), permettant ou bloquant respectivement la rotation angulaire d'une moitié (13) par rapport à l'autre (14), c'est-à-dire d'une barre (11) par rapport à l'autre (12), en opposition avec un ou plusieurs ressorts (24), **caractérisée en ce que** la première moitié (13) est pourvue d'une rainure semi-circulaire (16) et d'un siège (17) disposé dans une direction radiale, tandis que la seconde moitié (14) est pourvue d'une rainure (18) qui délimite une première dent (19), elle-même adaptée à définir un siège (20) qui, en utilisation, est disposé sur le même axe que le siège (17) présent dans la première moitié (13), et une seconde dent (21) adaptée à définir un siège (22).

2. Articulation (10) pour orthèses ou attelles orthopédiques selon la revendication 1, **caractérisée en ce qu'**elle comprend un sélecteur coulissant (23) insérable dans ledit siège (17) de manière à intercepter ledit siège (22) de la seconde moitié (14).

3. Articulation (10) pour orthèses ou attelles orthopédiques selon l'une des revendications 1 et 2, **caractérisé en ce que** ledit sélecteur coulissant (23) est pourvu d'une tige (25) adaptée à être insérée, en utilisation, dans ledit siège (17) de ladite première moitié (13) positionné en alignement avec ledit siège (20) de la seconde moitié (14).

4. Articulation (10) pour orthèses ou attelles orthopédiques selon l'une des revendications 1 à 3, **caractérisé en ce qu'**elle comprend au moins un ressort (24) insérable dans un logement (26) ménagé à partir d'une extrémité de ladite rainure semi-circulaire (16) de la première moitié (13).

5. Articulation (10) pour orthèses ou attelles orthopédiques selon l'une des revendications 1 à 4, **caractérisée en ce que** ledit sélecteur (23) peut être déplacé de manière à être amené d'une position ouverte à une position fermée par rapport audit siège (20), afin de permettre ou d'empêcher, respectivement, la rotation angulaire de la seconde barre (12) par rapport à la première barre (11).

6. Articulation (10) pour orthèses ou attelles orthopédiques selon l'une des revendications 1 à 5, **caractérisée en ce que**, lorsque le sélecteur (23) est en position ouverte, il demeure relevé par rapport audit siège (20) de la seconde moitié (14), permettant la rotation libre l'une par rapport à l'autre des deux moitiés (13, 14), et en conséquence des deux barres (11, 12).

7. Articulation (10) pour orthèses ou attelles orthopédiques selon l'une des revendications 1 à 6, **caractérisée en ce que** le sélecteur (23) peut être déplacé en position fermée dans le siège (17), pénétrant dans celuici jusqu'à ce qu'il atteigne le siège (20) de la seconde moitié (14), le verrouillage étant obtenu grâce à l'opposition entre le sélecteur (23) et la dent (19) qui empêche le mouvement angulaire entre les deux moitiés.

8. Articulation (10) pour orthèses ou attelles orthopédiques selon l'une des revendications 1 à 7, **caractérisée en ce que** la rotation entre les deux moitiés (13, 14), c'est-à-dire entre les deux barres (11, 12), est de type élastique, dans la mesure où elle s'effectue en opposition à la poussée exercée par un ou plusieurs ressorts (24) .
